# EUROPEAN PATENT SPECIFICATION

(11) **EP 3 082 974 B1**
(45) Date of publication and mention of the grant of the patent: **05.02.2020**
(21) Application number: 14833312.3
(22) Date of filing: 19.12.2014
(51) Int. Cl.: A61Q 17/04, A61Q 19/08, A61K 8/891, A61K 8/894, A61K 8/06

(54) **CARRIER SYSTEM FOR WATER-SOLUBLE ACTIVE INGREDIENTS**
TRÄGERSYSTEM FÜR WASSERLÖSLICHE WIRKSTOFFE
SYSTÈME DE TRANSPORTEUR D'INGRÉDIENTS ACTIFS HYDROSOLUBLES

(30) Priority: 20.12.2013 US 201314136634; 20.12.2013 US 201314136562
(43) Date of publication of application: 26.10.2016
(73) Proprietor: L'OREAL, 75008 Paris (FR)
(72) Inventor: CHIOU, Catherine, Saddle Brook, NJ 07663 (US); PAN, Zhi, Ridgewood, NJ 07450 (US); GALDI, Angelike, Westfield, NJ 07090 (US); MANNING, Lauren, Hoboken, NJ 07030 (US); GOLDBERG, Andrew, B., Springfield, NJ 07081 (US)
(74) Representative: Prinz & Partner mbB
(86) International application number: PCT/US2014/071515
(87) International publication number: WO 2015/095711

(56) References cited:
- EP-A2- 2 016 932
- EP-A2- 2 319 484
- WO-A1-00/24365
- WO-A2-2013/192004
- DE-A1-102009 045 981
- US-A1- 2007 128 137
- US-A1- 2007 264 210

## Description

### FIELD OF THE INVENTION

The present invention is directed to compositions containing water-soluble UV sunscreen filters. More specifically, the present invention is directed to a sunscreen composition in the form of an inverse water-in-silicone emulsion containing high levels of water-soluble UV filters. The composition is capable of carrying high amounts of water-soluble ingredients in a texturally pleasing manner, without experiencing separation of the emulsion. The present invention also provides a water-releasing effect when applied onto a keratinous substrate such as skin, hair or nails. The water-releasing effect enables the composition, initially in the form of an emulsion, to be converted into a plurality of droplets upon application of shear such as, for example, rubbing.

### BACKGROUND OF THE INVENTION

The incorporation of water-soluble active ingredients into emulsion-type compositions has posed various stability challenges. This has been especially true when the desire was to incorporate larger amounts of said ingredients into compositions. Examples of lack of stability include discoloration of the formula and/or precipitation of the ingredients out of the composition.

The photoprotection of keratinous substrates, especially skin, is considered by many to be necessary in order to facilitate protection from sunburn and photo-aging, as well as to decrease the chances of skin cancer development. There are typically two types of UV sunscreens used to accomplish photoprotection, namely, inorganic UV filters and organic UV filters.

Inorganic UV filters such as titanium dioxide and zinc oxide are typically employed in large quantities in order to ensure proper coverage/maximum protection over the surface onto which they are applied. As a result, they have a tendency to feel dry and impart an undesirable white color onto the treated surface.

Organic UV filters are typically classified as being either water-soluble or oil-soluble, based on their solubility. Oil-soluble UV filters, such as homosalate, octocrylene and avobenzone, while easy to incorporate into emulsions, often impart a greasy and tacky feeling onto a user's skin which consumers consider unpleasant and hence, undesirable.

Water-soluble UV filters do not impart this unpleasant feeling as they are considered by consumers to provide a much lighter skin feel. Water-soluble UV filters are often used in the form of a sodium salt (i.e., an electrolyte) in order to improve their solubility profile. The problem, however, is that such filters, due to their electrolytic properties, are difficult to formulate with when it comes to long-term stability. This lack of stability oftentimes manifests itself in the form of re-crystallization of the filters in the composition, causing them to separate from the emulsion. Additionally, water-soluble UV filters tend to have a detrimental effect on any thickener ingredients conventionally found in topical products as they too have a tendency to separate from the emulsion due to the electrolytic properties of the UV filters. As a result, water-soluble UV filters present a challenge for incorporation into emulsions intended for topical application onto a keratinous substrate, as most traditional emulsions are thickened and/or stabilized with natural or synthetic polymers such as gums and polyacrylates, which are very sensitive to electrolytes.

US 2007/0264210 A1 refers to a method of enhancing the delivery of water-soluble skin care actives into keratinous tissue, comprising the step of applying to the keratinous tissue a water-in-oil emulsion comprising an aqueous phase and a non-aqueous phase, wherein the aqueous phase comprises a water-soluble skin care active, and whereupon application of shear stress to the composition, the aqueous phase is visibly separated from the non-aqueous phase. The compositions comprise dimethicone and an emulsifying crosslinked siloxane elastormer.

US 2007/0128137 A1 discloses water in oil emulsion compositions comprising: from about 0.1 % to about 15% of a non-emulsifying crosslinked siloxane elastomer; from about 0.1% to about 15% of an emulsifying crosslinked siloxane elastomer; from about 1 % to about 40% of a solvent for the non-emulsifying and emulsifying crosslinked silox-ane elastomers; optionally, from 0% to about 5% of an additional emulsifier; and from about 50% to about 99% of aqueous phase; wherein when shear stress is applied to the composition during spreading on skin, aqueous phase is released from the emulsion.

WO 2013/192004 A2 is a post-published document and discloses a water-releasing cosmetic composition in the form of an emulsion and a process for preparing the cosmetic composition. The cosmetic composition includes an aqueous phase and an oil phase. The aqueous phase includes a hydrating agent at a concentration, by weight, of about 10% to about 50%, based upon weight of the composition. Tue oil phase includes dimethicone at concentration, by weight of about 1 % to about 25%, based upon weight of the composition. The oil phase include an emulsifying crosslinked siloxane elastomer at a concentration, by weight, of about 0.5% to about 7%, based upon weight of the composition. The cosmetic composition has a phase ratio of the aqueous phase to the oil phase of about 3.0 to about 12.0. The cosmetic composition converts from an emulsion to a plurality of droplets upon rubbing.

Other cosmetic compositions are shown in DE 10 2009 045981A1, EP 2 016 932 A2, WO 00/24365A1 and EP 2 319 484 A2.

It is therefore an object of the present invention to provide a composition capable of stably carrying water-soluble UV sunscreen filters, which is also tactilely pleasing to consumers upon application. In addition, it is an object of the present invention to provide a photoprotection composition containing at least one water-soluble UV filter, in a salt or an acid form, having improved stability but without having a greasy and/or tacky feel.

### BRIEF DESCRIPTION OF THE INVENTION

All numbers expressing quantities of ingredients and/or reaction conditions are understood as being modified in all instances by the term "about", unless otherwise stated.

The invention provides a composition in accordance with claim 1. In an exemplary embodiment, a composition in the form of a stable, tactilely pleasing emulsion containing significant amounts of at least one water-soluble active ingredient is provided. The at least one water-soluble acitve ingredient is a UV sunscreen filter. The composition includes an aqueous phase and an oil phase. The aqueous phase contains the at least one water-soluble ingredient at a concentration of from about 0.1% to about 20% by weight, based upon the total weight of the composition. The oil phase consists of dimethicone at a concentration by weight of from about 1% to about 25%, based upon the total weight of the composition, an emulsifying crosslinked siloxane elastomer at a concentration by weight of from about 0.1% to about 20%, based upon the total weight of the composition, and optionally a co-emulsifier at a concentration, by weight, of 0.01 to 1%, based upon the weight of the composition. The skin care composition has a phase ratio of aqueous phase to oil phase of from about 5.0 to about 9.0. The composition converts from an emulsion to a plurality of droplets upon application of shear such as, for example, rubbing with one's fingers or using an electromechanical force-imparting device such as, for example, an electromechanical cleansing brush.

In general, the present invention is directed to an inverse water-in-oil (silicone) emulsion type photoprotection composition containing an emulsifying crosslinked siloxane elastomer and water-soluble UV filters (WSUVF).

In another exemplary embodiment, a method of delivering skin care active ingredients on keratinous substrates is provided. The method includes applying the above-disclosed composition onto the surface of a keratinous substrate, followed by application of shear onto the composition present on the keratinous substrate.

of the composition can be used for inhibiting UV rays from contacting keratinous substrates. The use includes applying the above-disclosed photoprotection composition onto the surface of a keratinous substrate, followed by application of force onto the composition present on the keratinous substrate.

It has been surprisingly discovered by the inventors that the stability problems encountered by the use of water-soluble UV filters in emulsions can be mitigated by the use of an inverse water-in-oil emulsion, where the WSUVFs are incorporated into the dominant water phase prior to emulsification. Such an approach fully solubilizes the WSUVFs and their salts and provides the emulsion stability without the use of any water phase thickeners. Furthermore, the oil (silicone) external phase gives the composition a pleasant, silky feel upon topical application.

Other features and advantages of the present invention will be apparent from the following more detailed description of the preferred embodiment which illustrates, by way of example, the principles of the invention.

### DETAILED DESCRIPTION OF THE INVENTION

"Keratinous substrate," as used herein, includes but is not limited to skin, hair, and nails.

"Force", as used herein, includes shear/friction produced by a rubbing motion of an end user's fingers, an electromechanical cleansing device having a movable brush with bristles, and/or an electromechanical device that produces a tapping motion, similar to one's fingers tapping on the surface of the skin.

"Homogenous" means substantially uniform throughout, i.e., a single phase mixture.

In the present application the term "ambient temperature" means a temperature of about 25° C.

In the present application the term "stable" means the emulsion remains intact without phase separation, color and/or odor change over the stability monitoring period and the water-soluble active ingredients remain solubilized in the water phase without crystallization or precipitation out of the emulsion.

In the present application the term "water-releasing," as used herein, describes the phenomenon wherein, after application of a composition onto a target substrate, force is then applied onto the composition causing the water-in-oil type emulsion to rupture, which in turn causes the internal aqueous phase containing the water-soluble ingredient(s) to emerge in the form of droplets.

The anti-aging composition, photoprotection composition and method of the present invention can comprise, consist of, or consist essentially of the essential elements and limitations of the invention described herein, as well as any additional or optional ingredients, components, or limitations described herein or otherwise useful in compositions intended for topical application onto keratinous substrates.

It has been surprisingly discovered by the inventors that high concentrations of water-soluble active ingredients such as, for example, phenolic or polyphenolic acids, can be formulated into a stable water-in-oil type emulsion which, though initially in the form of a cream, possesses a transformative water-releasing effect upon application of force such as, for example, shear. The transformative water-releasing effect is that the cream transforms into droplets containing the aqueous phase with the water-soluble ingredient which, when exposed to force such as the shearing effect caused by rubbing/massaging the emulsion present on the surface of the keratinous substrate, thereby forcing the water-soluble ingredient-containing droplets into the keratinous substrate.

One advantage of an embodiment of the present disclosure includes providing a stable composition or stable photoprotection composition capable of carrying relatively high levels of electrolytic water-soluble ingredients without undergoing phase separation, i.e. recrystallizing and/or breaking the emulsion. Yet another advantage of an embodiment of the present disclosure is providing a skin care, cosmetic composition, or photoprotection composition capable of producing a water-releasing effect onto a keratinous substrate, such as skin. The water-releasing effect enables the composition, initially in the form of an emulsion, to be converted into a plurality of droplets carrying high levels of water-soluble ingredients upon application of force such as, for example, shear caused by an end user's rubbing of the composition onto the surface of a target keratinous substrate. The droplets, in turn, enable the water-soluble ingredients present in said droplets to effectively penetrate into a target keratinous substrate.

The water-in-oil emulsion system of the present invention typically has a white, glossy cream appearance. However, it may be modified so as to have a transparent gel-like or matte appearance by adjusting its refractive index. When the composition is deposited onto a target keratinous substrate, followed by application of force, the composition quickly releases the aqueous phase containing the water-soluble ingredients in the form of bead-like droplets, thereby enabling the water-soluble ingredients present in the aqueous phase to be forced into the surface of the target keratinous substrate. Likewise, when the photoprotection composition is deposited onto a target keratinous substrate, followed by application of force, the composition quickly releases the aqueous phase containing the water-soluble UV filters in the form of bead-like droplets, thereby enabling the water-soluble UV filters present in the aqueous phase to be spread onto the surface of the target keratinous substrate.

### Aqueous Phase

In one embodiment, the aqueous phase present in the composition according to the disclosure includes at least one water-soluble UV sunscreen filter, water, and other aqueous phase ingredients. The aqueous phase of the composition is at a concentration, by weight, of from about 60% to about 92%, or alternatively from about 70% to about 90%, or alternatively from about 80% to about 90% based upon weight of the composition.

In one embodiment, the aqueous phase present in the photoprotection composition according to the disclosure includes at least one water-soluble UV filter, water, and other aqueous phase ingredients. The aqueous phase of the photoprotection composition is at a concentration by weight of from about 60% to about 92%, or alternatively from about 70% to about 90%, or alternatively from about 80% to about 90%, based upon the weight of the photoprotection composition

### Water-Soluble Active Ingredient

In one embodiment, the aqueous phase present in the composition according to the disclosure includes at least one water-soluble UV sunscreen filter at a concentration, by weight, of from about 0.1% to about 20%, or alternatively from about 0.1% to about 15%, or alternatively from about 0.5% to about 10% based upon weight of the composition.

In one embodiment, the aqueous phase present in the photoprotection composition according to the disclosure includes at least one water-soluble UV filter at a concentration by weight of from about 2% to about 25% by weight, such as from about 3% to about 20% by weight, such as from about 4% to about 18% by weight, such as from about 4% to about 15% by weight, such as from about 5% to about 10% by weight, all weights based upon the weight of the composition.

In one embodiment, where the composition includes ellagic acid, the preferred concentration of ellagic acid, by weight, is from about 0.001% to about 0.01%, due to the inherently low water solubility of ellagic acid.

Suitable examples of water-soluble UV filters that may be used include, but are not limited to, terephthalylidene dicamphor sulfonic acid (Ecamsule), phenylbenzimidazole sulfonic acid (Ensulizole), Benzophenone-4, aminobenzoic acid (PABA), 4-Bis(polyethoxy)-para-aminobenzoic acid polyethoxyethyl ester (PEG-25 PABA), camphor benzalkonium methosulfate, methylene bis-benzotriazolyl tetramethylbutylphenol (Bisoctrizole), disodium phenyl dibenzimidazole tetrasulfonate (Bisdisulizole disodium), and tris-biphenyl triazine; their derivatives and corresponding salts; naphthalene bisimide derivatives such as those described in European patent application EP1990372 A2, the entire contents of which is hereby incorporated by reference; and cinnamido amine cationic quaternary salts and derivatives such as those described in United States Patent 5,601,811, the entire contents of which is hereby incorporated by reference, and mixtures thereof.

Other water-soluble active ingredients can be present in their synthetic chemical compound forms, or alternatively as integral part of botanical extracts. Suitable examples of water-soluble ingredients, include, but not limited to, (1) phenolic and polyphenolic compounds, and (2) other non-phenolic compounds.

The salts of the compounds that may be used according to the invention are chosen in particular from salts of alkali metals, for example sodium or potassium; salts of alkaline-earth metals, for example calcium, magnesium or strontium; metal salts, for example zinc, aluminium, manganese or copper; salts of ammonium of formula NH4⁺; quaternary ammonium salts; salts of organic amines, for instance salts of methylamine, dimethylamine, trimethylamine, triethylamine, ethylamine, 2-hydroxyethylamine, bis(2-hydroxyethyl)amine or tris(2-hydroxyethyl)amine; lysine or arginine salts. Salts chosen from sodium, potassium, magnesium, strontium, copper, manganese or zinc salts are preferably used. The sodium salt is preferentially used.

Phenolic and polyphenolic compounds include, but not limited to, flavones, chalcones, tannins, phenolic acids, catechins, anthocyanidins, stilbenoids, curcuminoids, phenylpropanenoids. Many of phenolic and polyphenolic compounds are well-known antioxidants and/or compounds that can provide skin care and cosmetic benefits. Some popular examples are baicalin, resveratrol, ferulic acid, ellagic acid, salicylic acid, and botanical extracts.

In general, any WSUVF capable of absorbing UV light in the range of from about 280 to about 400 nm can be employed in the present invention.

Other non-phenolic, water-soluble compounds include, but are not limited to, vitamins, xanthines, ceramides, cholesterols, sphingosines, C-glycosides, zwitterionic N-substituted amino sulfonic acid buffers, sugars, nucleic acids, α- and β-hydroxy acids, botanical extracts, aminopropyl triethoxysilane (APTES), dihydroxyacetone (DHA), amino acids, and peptides, and their derivatives and mixtures thereof.

### Optional Hydrotropes

The composition of the present disclosure may optionally include hydrotropes. Examples of suitable hydrotropes include, for example, but not limited to, nicotinamide, caffeine, sodium PCA, sodium salicylate, urea, or hydroxyethyl urea. At least one or a combination of two or more hydrotropes can be used to improve the solubility of phenolic and polyphenolic compounds in the water phase.

Hydrotropes may be present in the compositions in amounts generally ranging from about 0.1% to about 20% by weight, preferably from about 0.5% to about 10% by weight, and most preferably from about 1% to about 5% by weight, based on the total weight of the composition.

### Water

The aqueous phase present in the composition or photoprotection composition according to the disclosure includes water at a concentration by weight of about 60% to about 92%, or alternatively about 70% to about 90% or alternatively about 80% to about 90%, based upon the total weight of the composition. The water used may be sterile demineralized water and/or a floral water such as rose water, cornflower water, chamomile water or lime water, and/or a natural thermal or mineral water such as, for example: water from Vittel, water from the Vichy basin, water from Uriage, water from La Roche Posay, water from La Bourboule, water from Enghien-les-Bains, water from Saint Gervais-les-Bains, water from Neris-les-Bains, water from Allevar-les-Bains, water from Digne, water from Maizieres, water from Neyrac-les-Bains, water from Lons-le-Saunier, water from Eaux Bonnes, water from Rochefort, water from Saint Christau, water from Les Fumades, water from Tercis-les-Bains or water from Avene. The water phase may also comprise reconstituted thermal water, that is to say a water comprising trace elements such as zinc, copper, magnesium, etc., reconstituting the characteristics of a thermal water.

### Oil Phase

The oil phase present in the composition or photoprotection composition according to the disclosure consists of dimethicone, an emulsifying crosslinked siloxane elastomer and, optionally, a co-emulsifyier. The oil phase of the water-releasing composition is at a concentration by weight of about 8% to about 25%, or alternatively about 10% to about 20%, or alternatively about 10% to about 15%, based upon the total weight of the composition.

### Dimethicone

The oil phase present in the composition or photoprotection composition according to the disclosure includes dimethicone at a concentration, by weight, of about 1% to about 25%, or alternatively about 2% to about 20%, or alternatively about 4% to about 15%, based upon weight of the composition.

### Emulsifying Crosslinked Siloxane Elastomer

The oil phase present in the composition or photoprotection composition according to the disclosure includes an emulsifying crosslinked siloxane elastomer at a concentration, by weight, of about 0.1% to about 20%, or alternatively about 0.3% to about 10%, or alternatively about 0.5% to about 7%, based upon weight of the composition.

Examples of suitable emulsifying crosslinked siloxane elastomers, include, but are not limited to, substituted or unsubstituted dimethicone/copolyol crosspolymer, dimethicone and dimethicone/PEG-10/15 crosspolymers, substituted or unsubstituted dimethicone/polyglyceryl crosspolymer, dimethicone and dimethicone/polyglycerin-3 crosspolymer. Such suitable emulsifying crosslinked siloxane elastomers are sold or made, for example, under the names of "KSG-210" a polyether-modified cross polymer with an INCI name of dimethicone (and) dimethicone/PEG-10/15 crosspolymer, and "KSG-710" a polyglycerin-modified crosspolymer with an INCI name of dimethicone (and) dimethicone/polyglycerin-3 crosspolymer, both available from Shin-Etsu Silicones of America, Inc. (Akron, OH).

### Co-emulsifier

In one embodiment, the oil phase present in the composition according to the disclosure may optionally include a co-emulsifier at a concentration by weight of about 0.01% to about 1%, or alternatively about 0.05% to about 0.9%, or alternatively about 0.07% to about 0.8%, based upon the total weight of the composition. If the co-emulsifier concentration exceeds 1% by weight of the anti-aging composition, then the composition may still form an emulsion but the desirable transformative effect of cream changing to droplets upon application of shear is not achieved.

Suitable examples of co-emulsifiers include polyether substituted linear or branched polysiloxane copolymers. One preferred co-emulsifier is PEG-10 dimethicone available under the tradename of ES-5612 from Dow Corning Corporation (Midland, Michigan), or KF-6017 from Shin-Etsu (Akron, Ohio). Another preferred co-emulsifier is dimethicone (and) PEG/PPG-18/18 dimethicone available under the tradename of ES-5226 DM from Dow Corning Corporation (Midland, Michigan). Other suitable co-emulsifiers include, PEG-9 polydimethylsiloxyethyl dimethicone available under the tradename KF-6028 and PEG-9,lauryl PEG-9 polydimethylsiloxyethyl dimethicone available under the tradename KF-6038, both available from Shin-Etsu (Akron, Ohio). Another suitable example of a co-emulsifier is polyoxyalkylene copolymers also known as silicone polyethers. Polyoxyalkylene copolymers are described in detail in U.S. Pat. 4,268,499, which is incorporated herein by reference in its entirety. A particularly preferred polyoxyalkylene copolymer is known by its CTFA designation as dimethicone copolyol. A particularly preferred form of dimethicone copolyol is supplied by Dow Corning as DC5225C.

### Optional Powders

The composition or photoprotection composition of the present disclosure may optionally include powders. The optional powders provide formulas that are smoother and softer on the skin. Representative powders include, but are not limited to talc, mica, magnesium carbonate, calcium carbonate, magnesium silicate, aluminum magnesium silicate, silica, titanium dioxide, zinc oxide, red iron oxide, yellow iron oxide, black iron oxide, polyethylene powder, methacrylate powder, polystyrene powder, silk powder, crystalline cellulose, starch, titanated mica, iron oxide titanated mica, bismuth oxychloride, and the like. Additional powders include, but are not limited to, inorganic powders such as gums, chalk, Fuller's earth, kaolin, sericite, muscovite, phlogopite, synthetic mica, lepidolite, biotite, lithia mica, vermiculite, aluminum silicate, starch, smectite clays, alkyl and/or trialkyl aryl ammonium smectites, chemically modified magnesium aluminum silicate, organically modified montmorillonite clay, hydrated aluminum silicate, fumed aluminum starch octenyl succinate barium silicate, calcium silicate, magnesium silicate, strontium silicate, metal tungstate, magnesium, silica alumina, zeolite, barium sulfate, calcined calcium sulfate (calcined gypsum), calcium phosphate, fluorine apatite, hydroxyapatite, ceramic powder, metallic soap (zinc stearate, magnesiumstearate, zinc myristate, calcium palmitate, and aluminum stearate), colloidal silicone dioxide, and boron nitride; organic powder such as polyamide resin powder (nylon powder), cyclodextrin, methyl polymethacrylate powder, copolymer powder of styrene and acrylic acid, benzoguanamine resin powder, poly(ethylene tetrafluoride) powder, and carboxyvinyl polymer, cellulose powder such as hydroxyethyl cellulose and sodium carboxymethyl cellulose, ethylene glycol monostearate; inorganic white pigments such as magnesium oxide. A representative powder includes, for example, polymethylsilsesquioxane. Powders may be present in the compositions in amounts generally ranging from about 0.1% to about 5% by weight or about 0.1% to about 10% by weight, based on the total weight of the composition.

### Phase Ratio

The phase ratio is calculated by dividing the total weight of the aqueous phase by the total weight of the oil phase. The anti-aging composition or photoprotection composition of the present disclosure as a water-in-oil emulsion has a ratio by weight of the aqueous phase to oil phase of from about 5 to about 9. The phase ratio excludes any additional optional powders that may be added to the composition. Without intending to be bound by theory, this phase ratio is believed to be critical to: (1) the stability of the emulsion in view of the high concentration of water-soluble ingredient(s) contained therein, and (2) the formation of droplets upon application of force onto the emulsion.

### Water-Releasing Effect

With respect to the present invention, a good water-releasing effect of the water-in-oil emulsion means that the water-releasing effect has an evaluation result of more than or equal to a score of 3 in the evaluation system described below. The test method and evaluation score of the test system are described below.

About 0.2 g of a water-in-oil emulsion sample of the composition is taken and placed on the back of a hand, then it is applied thereon by circling gently with the middle finger and ring finger of the other hand, and then the phenomenon of the water-releasing effect is observed when the circling application reaches 20 cycles, and evaluated by a 5- level scoring system. A score of 5 represents that more than 10 bead-like water drops having an average diameter of more than or equal to 3 mm appear, or more than 20 bead-like water drops having an average diameter of more than or equal to 1 mm appear. A score of 4 represents that 2-10 bead-like water drops having an average diameter of more than or equal to 3 mm appear, or 10-20 bead-like water drops having an average diameter of more than or equal to 1 mm appear and the beadlike water drops having an average of more than or equal to 3 mm are no more than 10. A score of 3 represents that 2-9 bead-like water drops having an average diameter of more than or equal to 1 mm appear and there is at most 1 bead-like water drop having an average diameter of more than or equal to 3 mm, or 10-20 bead-like water drops having an average diameter of 1 mm appear. A score of 2 represents that 2-9 bead-like, water drops having an average diameter of 1 mm appear. A score of 1 represents that no water drop appears. Each level between scores 5 to 4, 4 to 3, 3 to 2, and 2 to 1 shows that the water-releasing effect is between the two end values described above, and the lower the score, the poorer the water-releasing effect.

In one embodiment, the water-releasing effect of the cosmetic composition of the present disclosure is about 3 to 5.

### EXAMPLES

The method of making each of the examples provided in Tables 1 and 2 is generally the same. The examples in Tables 1 and 2 include comparative examples of emulsions with incorporation of high concentrations of water-soluble active ingredients other than UV sunscreen filters having a water-releasing effect. The examples in Tables 3 and 4 are comparative examples illustrating the lack of emulsion stability and/or stabilization of active ingredients in a typical water-in-oil emulsion.

In each example (inventive and comparative), the viscosity of the emulsion is measured using a Brookfield Viscometer, using Heliopath spindle T-D and speed set at 10 rpm. The spindle was allowed to oscillate in the test sample, and the measurements were taken after one minute.

The water-releasing effect of each example is measured by placing about 0.2 g of the cosmetic composition on the back of a hand, then applying thereon by circling gently with the middle finger and ring finger of the other hand. The phenomenon of the water-releasing effect is observed when the circling application reaches 20 cycles.

Microscopic images of the emulsion were captured to monitor the stabilization of the water-soluble ingredients in the emulsion. The Microscope is set at 10 time magnification, using a Leica DM2500 microscope and analyzed with the Leica Application Suite software.

All examples were monitored for emulsion stability and the stabilization of active ingredients for a period of 8 weeks at 5°C, 25°C, 37°C and 45°C and after 10 cycles of freeze/thaw (ranged from -20°C to 25°C). Viscosity and microscopic pictures were taken at weeks 4 arid 8 by measuring at 25°C.

**Table 1. Comparative Examples**

| **Phase** | **INCI Name** | | **Ex.1** | **Ex.2** | **Ex.3** | **Ex.4** | **Ex.5** |
|---|---|---|---|---|---|---|---|
| A | DIMETHICONE/PEG-10/15 CROSSPOLYMER | | 1.25 | 1.25 | 1.25 | 1.25 | 0.75 |
| A | DIMEIHICONE/POLYGLYCERIN -3 CROSSPOLYMER | | | | | | 0.5 |
| A | PEG-10 DIMETHICONE | | 0.1 | 0.1 | 0.1 | 0.1 | 0.1 |
| A | DIMETHICONE (and) DIMEIHICONOL (88/12) | | 1 | 1 | 1 | 1 | 1 |
| A | DIMETHICONE | | 13.7 | 13.7 | 13.7 | 13.7 | 13.7 |
| B | WATER, PRESERVATIVES | | QS 100 | QS 100 | QS 100 | QS 100 | QS 100 |
| B | NIACINAMIDE | | 5 | | 5 | | |
| B | CAFFEINE | | 5 | | 5 | | |
| B | ENGELHARDTIA CHRYSOLEPIS LEAF EXTRACT | | 0.5 | | | | |
| B | FERULIC ACID | | 0.5 | | | | |
| B | 3-O-ETHYL ASCORBIC ACID | | | 1 | 5 | | |
| B | HYDROXYPHENOX PROPIONIC ACID | | | | 1 | | |
| B | ELLAGIC ACID | | | | 0.01 | | |
| B | CETEARETH-25 (and) | | | | | 0.14 | |
| | GLYCERIN (and) CETYL | | | | | | |
| | ALCOHOL (and) CERAMIDE | | | | | | |
| | NP (and) BEHENIC ACID | | | | | | |
| | (and) CHOLESTEROL(and) | | | | | | |
| | CERAMIDENS (and) | | | | | | |
| | CERAMIDE EOP (and) | | | | | | |
| | CERAMIDE EOS (and) | | | | | | |
| | CERAMIDE AP(and) | | | | | | |
| | CAPROOYL PHYTOSPHINGOSINE (and) | | | | | | |
| | CAPROOYL SPHINGOSINE | | | | | | |
| B | HYDROXYETHYLPIPERAZINE ETHANE SULFONIC ACID | | | | | 1 | |
| B | HYDROXYPROPYL TETRAHYDROPYRANTRIOL | | | | | 3.3 | |
| B | MANNOSE | | | | | | 10 |
| B | SALICYLIC ACID | | | | | | 2 |
| B | ALCOHOL DENAT. | | | | | | 5 |
| B | GLYCERIN | | 3 | 3 | 3 | 3 | 3 |
| B | SODIUM CITRATE | | 0.2 | 0.2 | 0.2 | 0.2 | 0.2 |
| B | SODIUM CHLORIDE | | 0.5 | 0.5 | 0.5 | 0.5 | 0.5 |
| B | CITRIC ACID | | | 0.2 | 0.2 | | 0.2 |
| C | POLYMETHYLSILSESQUIOXANE | | 1 | 1 | 1 | 1 | 1 |
| | Total (%) | | 100 | 100 | 100 | 100 | 100 |
| | Brookfield Viscosity (cp) | | 38,400 | 65,000 | 55,500 | 28,000 | 45,600 |
| | Total Oil Phase (%) | | 12.27 | 12.07 | 12.07 | 12.07 | 12.07 |
| | Total Water Phase (%) | | 86.43 | 86.93 | 86.93 | 86.93 | 86.93 |
| | Ratio (Water Phase/Oil Phase) | | 7.04 | 7.20 | 7.20 | 7.20 | 7.20 |
| | Water Releasing Effect | | 5 | 4 to 5 | 4 to 5 | 4 to 5 | 5 |
| | Texture/Appearance | | Translucent Water dro to opaque, plets released gel-like cream. upon rubbing. | | | | |
| | Stability Results | Emulsion remained stable after 8 weeks. No crystals formed. | | | | | |

**Table 2. Comparative Examples**

| **Phase** | **INCI Name** | **Ex.6** | **Ex.7** | **Ex.8** | **Ex.9** | **Ex.10** |
|---|---|---|---|---|---|---|
| A | DIMETHICONE/PEG-10/15 CROSSPOL YMER | 1.25 | 1.25 | 1.25 | 1.25 | 1.25 |
| A | PEG-10 DIMETHICONE | 0.1 | | 0.1 | | 0.07 |
| A | LAURYL PEG-9 POLYDIMETHYLSILOXYETHYL DIMETHICONE | | 0.1 | | 0.1 | |
| A | DIMETHICONE | 10.7 | 10.7 | 10.7 | 10.7 | 10.7 |
| A | TOCOPHEROL | 0.2 | | | | |
| B | WATER, PRESERVATIVES | QS 100 | QS 100 | QS 100 | QS 100 | QS 100 |
| B | ADENOSINE | 0.6 | | | | |
| B | SCUTELLARIA BAICALENSIS ROOT EXTRACT | | 0.1 | 0.5 | 0.5 | |
| B | CAFFEINE | 1 | 1 | 5 | | |
| B | NIACINAMIDE | 1 | 1 | 5 | | |
| B | CAPRYLOYL SALICYLIC ACID | 0.1 | 0.1 | 0.1 | 0.1 | |
| B | RESVERATROL | | | | 0.5 | |
| B | GLYCERIN | 15 | 15 | 15 | 15 | 3 |
| B | DIHYDROXYACETONE | | | | | 5 |
| B | PROPANEDIOL | 5 | 5 | | 5 | |
| B | PROPYLENE GLYCOL | | | 5 | | |
| B | DISODIUM EDTA | 0.1 | 0.1 | 0.1 | 0.1 | 0.1 |
| B | SODIUM CITRATE | 0.2 | 0.2 | 0.2 | 0.2 | 0.2 |
| B | SODIUM CHLORIDE | 0.8 | 0.8 | 0.8 | 0.8 | 0.5 |
| B | ALCOHOL DENAT. | 3 | 3 | 3 | 3 | |
| B | CITRIC ACID | | | | | 0.2 |
| B | CARAMEL | | | | | 0.03 |
| B | RED 4 | | | | | 0.0002 |
| C | SILICA SILYATE | 0.7 | 0.5 | 0.5 | 0.5 | |
| | Total (%) | 100 | 100 | 100 | 100 | 100 |
| | Brookfield Viscosity (cp) | 38,500 | 37,000 | 35,000 | 36,000 | 53,600 |
| | Total Oil Phase (%) | 16.8 | 16.6 | 16.6 | 16.6 | 12.07 |
| | Total Water Phase (%) | 83.2 | 83.4 | 8.4 | 83.4 | 87.93 |
| | Ratio (Water Phase/Oil Phase) | 4.50 | 5.02 | 5.02 | 5.02 | 7.29 |
| | Water Releasing Effect | 4 | 3 to 4 | 4 | 3 to 4 | 4 to 5 |
| | Texture/Appearance | Translucent to opaque, gel-like cream. Water droplets released upon rubbing. | | | | |
| | Stability Results | Emulsion remained stable after 8 weeks. No crystals formed. | | | | |

In making each of the inventive examples in Table 1 and 2, the following procedure is used. The ingredients of Phase A (oil phase) were placed in a main beaker and were mixed well with a propeller mixer at about 600-700 RPM and set aside. The ingredients of Phase B (aqueous) were mixed together in a side beaker with a propeller mixer at about 600-700 RPM until all solids were dissolved, giving a clear solution. If needed, Phase B (aqueous) was gently heated to about 40-45°C until all solids were dissolved. The mixture of aqueous phase ingredients (Phase B) was slowly added to the mixed ingredients of Phase A (oil phase) using a propeller mixer over a period of 10-15 minutes for an about 1 kg batch. As the viscosity of the mixture increased, the stirring speed was increased from 700 rpm to about 1200 rpm. As the aqueous phase is mixed into the oil phase a water-in-oil emulsion was formed. The powders of phase C were added to the batch and were mixed into the water-in-oil emulsion.

**Table 3. Comparative Example**

| **Phase** | **INCI Name** | **Ex. 11** |
|---|---|---|
| A | PEG/PPG-18/18 DIMETHICONE | 1.75 |
| A | DIMETHICONE | 20.75 |
| B | WATER, PRESERVATIVES | QS 100 |
| B | SCUTELLARIA BAICALENSIS ROOT EXTRACT | 0.4 |
| B | CAFFEINE | 5 |
| B | NIACINAMIDE | 5 |
| B | GLYCERIN | 10 |
| B | PROPYLENE GLYCOL | 10 |
| B | DIPROPYLENE GLYCOL | 10 |
| B | DISODIUM EDTA | 0.1 |
| B | SODIUM CHLORIDE | 2 |
| C | ALCOHOL DENAT. | 5 |
| Total | Total (%) | 100 |
| | Brookfield Viscosity (cp) | 66,000 |
| | Total Oil Phase (%) | 22.5 |
| | Total Water Phase (%) | 77.5 |
| | Ratio (Water Phase/Oil Phase) | 3.44 |
| | Water Releasing Effect | 1 |
| | Texture/Appearance | Light yellow, translucent cream-gel. No waterdroplets released upon rubbing. |
| Stability Results | | Emulsion remained stable after 8 weeks, but needle-shaped crystals formed on surface of the emulsion. |

In making Example 11 in Table 3, the following procedure is used. The ingredients of Phase A (oil phase) were placed in a main beaker and were mixed well with a propeller mixer at about 600-700 RPM and set aside. The ingredients of Phase B (aqueous) were mixed together in a side beaker with a propeller mixer at about 600-700 RPM until all solids were dissolved, giving a clear solution. If needed, Phase B (aqueous) was gently heated to about 40-45°C until all solids were dissolved. The mixture of aqueous phase ingredients (Phase B) was slowly added to the mixed ingredients of Phase A (oil phase) using a propeller mixer over a period of 10-15 minutes for an about 1 kg batch. As the viscosity of the mixture increased, the stirring speed was increased from 700 rpm to about 1200 rpm. As the aqueous phase is mixed into the oil phase, a water-in-oil emulsion was formed. Lastly, alcohol (Phase C) was added to the emulsion.

**Table 4. Comparative Example**

| **Phase** | **INCI Name** | **Ex. 12** |
|---|---|---|
| A | DIMETHICONE/PEG-10/15 CROSSPOLYMER | 1 |
| A | DIMETHICONE (and) | 1 |
| | DIMEIHICONOL(88/12) | |
| A | DIMETHICONE | 9 |
| A | TRISILOXANE | 16 |
| B | WATER | QS 100 |
| B | PHENOXYETHANOL | 0.6 |
| B | CAPRYLYL GLYCOL | 0.2 |
| B | HEXYLENE GLYCOL | 0.1 |
| B | IODOPROPYL CARBAMATE | 0.01 |
| B | POLYAMINOPROPYL BIGUANIDE | 0.04 |
| B | BUTYLENE GLYCOL | 2 |
| B | GLYCERIN | 10 |
| B | SODIUM CITRATE | 0.5 |
| | Total (%) | 100 |
| | Brookfield Viscosity (cp) | 5,000 |
| | Total Oil Phase (%) | 27 |
| | Total Water Phase (%) | 73 |
| | Ratio (Water Phase/Oil Phase) | 2.70 |
| | Water Releasing Effect | 1 |
| Texture/Appearance | | Translucent, milky serum. Watery on skin upon application. No water droplets released upon rubbing. |
| Stability Results | | Emulsion separated after 3 days of freeze-thaw cycles |

In making Example 12 in Table 4, the following procedure is used. The ingredients of Phase A (oil phase) were placed in a main beaker and were mixed well with a propeller mixer at about 600-700 RPM and set aside. The ingredients of Phase B (aqueous) were mixed together in a side beaker with a magnetic stirring bar until all solids were dissolved, giving a hazy solution. The mixture of aqueous phase ingredients (Phase B) was slowly added to the mixed ingredients of Phase A (oil phase) using a propeller mixer over a period of 10-15 minutes for an about 1 kg batch. As the viscosity of the mixture increased, the stirring speed was increased from 700 rpm to about 1000 rpm. As the aqueous phase is mixed into the oil phase a water-in-oil emulsion was formed.

Comparative Example 12, in contrast to the present disclosure, had a total weight percentage of the aqueous phase or water phase of about 73% and a total weight percentage of oil of about 27%, making the ratio of the aqueous phase to oil phase about 2.7. Comparative Example 12 formed a translucent, milky serum that is watery on skin upon application. Comparative Example 12 has no water-releasing effect.

Comparative Example 12 is generally unstable. The microscopic image showed that the water-in-oil emulsion boundary had a leaking boarder, indicating potential instability of emulsion. Though the serum of comparative Example 12 initially formed as an emulsion, after 3 days of freeze-thaw cycles, the serum completely separated.

**Table 5. Inventive Examples 13-17**

| **Phase** | **INCI Name** | **Ex.13** | **Ex.14** | **Ex.15** | **Ex.16** | **Ex.17** |
|---|---|---|---|---|---|---|
| A | DIMETHICONE | 7 | 7 | 7 | 6 | 6 |
| A | DIMETHICONE (and) DIMETHICONE/PEG-10/15 CROSSPOLYMER (KSG-210) | 5 | 5 | | 6 | 6 |
| A | DIMETHICONE (and) DIMETHICONE/POLYGLYCERIN -3 CROSSPOLYMER (KSG-710) | | | 5 | | |
| B1 | WATER, PRESERVATIVES | 53.21 | 56.87 | 56.87 | 46.41 | 37.15 |
| B1 | GLYCERIN | 15 | 15 | 15 | 15 | 15 |
| B1 | PROPANEDIOL | 3 | 3 | 3 | 3 | 3 |
| B1 | SODIUM CHLORIDE | 0.5 | 0.5 | 0.5 | 0.5 | 0.5 |
| B1 | SODIUM CITRATE | 0.2 | 0.2 | 0.2 | 0.2 | 0.2 |
| B1 | DISODIUM EDTA | 0.1 | 0.1 | 0.1 | 0.1 | 0.1 |
| B1 | SODIUM HYDROXIDE | 0.05 | 0.05 | 0.05 | 0.05 | 0.05 |
| B2 | TEREPHTHALYLIDENE DICAMPHOR SULFONIC ACID (ECAMSULE) | 3 | | | 3 | |
| B2 | BENZOPHENONE-4 | | | | | 5 |
| B2 | PHENYLBENZIMIDAZOLE SULFONIC ACID (ENSULIZOLE) | 3 | 4 | 4 | 5 | 4 |
| B2 | WATER | 7.2 | 7.2 | 7.2 | 12 | 20 |
| B2 | TRIETHANOLAMINE | 1.8 | 0 | 0 | 1.8 | 2.5 |
| B2 | SODIUM HYDROXIDE | 0.44 | 0.58 | 0.58 | 0.44 | |
| C | POLYMETHYLSILSESQUIOXANE | 0.5 | 0.5 | 0.5 | 0.5 | 0.5 |
| | Total (%) | 100 | 100 | 100 | 100 | 100 |
| | Brookfield Viscosity (cp) | 46,000 | 33,000 | 61,000 | 58,000 | 41,000 |
| | Total Oil Phase (%) | 12 | 12 | 12 | 12 | 12 |
| | Total Water Phase (%) | 88 | 88 | 88 | 88 | 88 |
| | Ratio (Water Phase/Oil Phase) | 7.33 | 7.33 | 7.33 | 7.33 | 7.33 |
| | Water Releasing Effect | 3 to 4 | 4 | 4 | 4 to 5 | 4 |
| | **Texture/Appearance:** | Translucent to opaque, light yellow to white, gel-like cream | | | | |
| **Stability Results:** | | Emulsions remained stable after 12 weeks. Nc crystals formed. | | | | |

In making each of the examples in Table 5, the following procedure is used.

The ingredients of Phase B (aqueous) are mixed together in a side beaker with a rotor/stator mixer until all solids are dissolved, giving a clear solution. If needed, Phase B (aqueous) can be gently heated to about 40-45°C until all solids are dissolved. The ingredients of Phase A (oil phase) are placed in a main beaker and are mixed well with a propeller mixer at about 600-700 RPM and set aside. The mixture of aqueous phase ingredients (Phase B) are slowly added to the mixed ingredients of Phase A (oil phase) using a propeller mixer over a period of 10-15 minutes for an about 1kg batch. As the viscosity of the mixture increases, the stirring speed is increased from 700 rpm to about 1200 rpm. As the aqueous phase is mixed into the oil phase a water-in- oil emulsion is formed. Optionally, powders are added to the batch and are mixed into the water-in-oil emulsion.

**Table 6. Example 18 (Comparative)**

| **Phase** | **US INCI Name** | **Ex. 18** |
|---|---|---|
| A | OCTOCRYLENE | 7 |
| A | ETHYLHEXYL SALICYLATE (OCTISALATE) | 5 |
| A | BUTYL METHOXYDIBENZOYLMETHANE (AVOBENZONE) | 3 |
| A | DIMETHICONE (and) DIMETHICONE/PEG-10/15 CROSSPOLYMER (KSG-210) | 5 |
| A | CETYL PEG/PPG-10/1 DIMETHICONE (ABIL EM 90) | 1 |
| B | WATER, PRESERVATIVES | 59.65 |
| B | GLYCERIN | 15 |
| B | PROPANEDIOL | 3 |
| B | SODIUM CHLORIDE | 0.5 |
| B | SODIUM CITRATE | 0.2 |
| B | DISODIUM EDTA | 0.1 |
| B | SODIUM HYDROXIDE | 0.05 |
| C | POLYMETHYLSILSESQUIOXANE(TOSPEARL 145 A) | 0.5 |
| | Total (%) | 100 |
| | Brookfield Viscosity (cp) | NA |
| | Total Oil Phase (%) | 21 |
| | Total Water Phase (%) | 79 |
| | Ratio (Water Phase/Oil Phase) *: | 3.76 |
| | Water Releasing Effect: | NA |
| | Texture/Appearance: | Thick, white cream |
| | Stability Results: | Not stable. Phase separated |

| | | |
|---|---|---|
| *Excludes powders (Phase C). | | |

The comparative Example 18 was prepared by heating water phase and oil phase separately to 80°C. The water phase was added to the oil phase while mixing until homogeneous. The resulted emulsion was cooled to about room temperature. Optionally, powders were added and mixed well.

Similar to the inventive examples 13-17, the water-in-oil emulsion of Example 18 used emulsifying siloxane elastomer (KSG-210) as the primary emulsifier and an alkyl-substituted polyether dimethicone copolymer (Abil EM90) as the co- emulsifier. Organic UV filters were added to the oil phase to make a sunscreen emulsion. Example 18 includes about 7% by weight octylcrylene, about 5% octisalate and about 3% avobenzone. The emulsion in Example 18 was formed initially as a thick, white cream, but its phases separated the following day. Thus, it was not possible to measure its "water-releasing" effect, nor its viscosity.

**Table 7. Example 19 (Comparative)**

| **Phase** | **INCI Name** | **Ex. 19** |
|---|---|---|
| A | Dimethicone (and) Dimethicone/PEG-10/15 Crosspolymer (KSG-210) | 4 |
| A | Dimethicone (and) Dimethiconol (88/12) | 1 |
| A | Dimethicone | 6 |
| A | Trisiloxane | 16 |
| B | Water | 59.3 |
| B | Phenoxyethanol | 0.6 |
| B | Caprylyl glycol | 0.2 |
| B | Hexylene gylcol | 0.1 |
| B | Iodopropynyl Butylcarbamate (10%) | 0.1 |
| B | Polyaminopropyl Biguanide (20% in water) | 0.2 |
| B | Butylene glycol | 2 |
| B | Glycerin | 10 |
| B | Sodium Citrate | 0.5 |
| | Total(%) | 100 |
| | Brookfield Viscosity (cp) | 5000 |
| | Total Oil Phase (%) | 27 |
| | Total Water Phase (%) | 73 |
| | Ratio (Water Phase/Oil Phase) | 2.7 |
| | Water Releasing Effect | 1 |
| | Texture: | |
| | Translucent, milky serum; watery on skin upon application. No "water-beading/releasing" effect. | |
| | Microscope: | |
| | Unstable. W/Si with leaking border, indicating potential instability of emulsion. | |

In making comparative Example 19, the following procedure was used. The ingredients of Phase B (aqueous) are mixed in together in a side breaker using a stirring bar to mix well and dissolve all solids. The ingredients of Phase A (oil phase) are placed in a main beaker and mixed well with a propeller mixer at about 600-700 RPM and set aside. The mixture of aqueous phase ingredients (Phase B) are slowly added to the mixed ingredients of Phase A using a prop mixer over a period of 10-15 minutes for a 1 kg batch. As viscosity slowly increased, the stirring speed is increased from 700 RPM to 1000 RPM to form a serum.

Comparative Example 19, in contrast to the present disclosure, has a total weight percentage of the aqueous phase or water phase of about 73% and a total weight percentage of oil of about 27%, making the ratio of the aqueous phase to oil phase about 2.7. Comparative Example 19 forms a translucent, milky serum that is watery on skin upon application. The viscosity of comparative Example 19 is measured using a Brookfield Viscometer, using spindle T-D and speed set at 10 rpm for 1 minute. The viscosity of comparative Example 19 is about 5,000 cp (mPas). The water-releasing effect of comparative Example 19 is measured by placing about 0.2 g of the cosmetic composition on the back of a hand, then applying thereon by circling gently with the middle finger and ring finger of the other hand. No bead-like droplets having an average diameter of more than or equal to 1 mm appeared. The water-releasing effect of the serum of comparative Example 19 is about 1; therefore, comparative Example 19 has no water- releasing effect.

Comparative Example 19 is generally unstable. The microscope shows that the W/Si boundary has a leaking border, indicating potential instability of emulsion. Though the serum of comparative Example 19 initially forms as an emulsion, after 3 days of freeze-thaw cycles, the serum completely separates.

While the invention has been described with reference to a preferred embodiment, it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment disclosed as the best mode contemplated for carrying out this invention, but that the invention will include all embodiments falling within the scope of the appended claims.

## Claims

1. A composition comprising:
an aqueous phase including at least one water-soluble UV sunscreen filter at a concentration by weight of from 2% to 25%, based upon weight of the composition; and
an oil phase consisting of:
dimethicone, at a concentration by weight of from 1% to 25%, based upon weight of the composition;
an emulsifying crosslinked siloxane elastomer at a concentration, by weight, of 0.1% to 20%, based upon weight of the composition; and
optionally, a co-emulsifier at a concentration, by weight, of 0.01% to 1%, based upon weight of the composition;
wherein a ratio, by weight, of the aqueous phase to the oil phase is 5.0 to 9.0; and
wherein the composition converts from an emulsion to a plurality of droplets upon application of shear.

2. The composition of claim 1, wherein the UV sunscreen filter is chosen from terephthalylidene dicamphor sulfonic acid (Ecamsule), phenylbenzimidazole sulfonic acid (Ensulizole), Benzophenone-4, aminobenzoic acid (PABA), camphor benzalkonium methosulfate, methylene bis-benzotriazolyl tetramethylbutylphenol (Bisoctrizole), disodium phenyl dibenzimidazole tetrasulfonate (Bisdisulizole disodium), tris-biphenyl triazine; their derivatives and corresponding salts; naphthaline bisimide derivatives, and cinnamido amine cationic quaternary salts and derivatives, and mixtures thereof.

3. The composition of claim 1, wherein the aqueous phase further includes an other water-soluble active ingredientchosen from flavones, stilbenoids, tannins, phenolic acids, polyphenolics, vitamins, xanthines, ceramides, cholesterols, sphingosines, C-glycosides, zwitterionic N-substituted amino sulfonic acid buffers, sugars, nucleic acids, α- and β-hydroxy acids, aminopropyl triethoxysilane, dihydroxyacetone, botanical extracts, amino acids, and peptides, their derivatives, and combinations thereof.

4. The composition of claim 1, wherein the composition further includes at least one hydrotrope selected from nicotinamide, caffeine, sodium PCA, sodium salicylate, urea, or hydroxyethyl urea.

5. The composition of claim 1, wherein the co-emulsifier is a polyoxyalkylene copolymer.

6. The composition of claim 5, wherein the co-emulsifier is chosen from PEG-10 dimethicone, PEG-9 polydimethylsiloxyethyl dimethicone and PEG-9, lauryl PEG-9 polydimethylsiloxyethyl dimethicone, dimethicone and PEG/PPG-18/18 dimethicone, and combinations thereof.

7. The composition of claim 1, further including a powder at a concentration by weight of from 0.1% to 5%, based upon weight of the composition.

8. The composition of claim 1 wherein:
(a) the aqueous phase includes at least one water- soluble UV sunscreen filter chosen from terephthalylidene dicamphor sulfonic acid (Ecamsule), phenylbenzimidazole sulfonic acid (Ensulizole), and Benzophenone-4, at a concentration by weight of from 4% to 10%, based upon weight of the composition, wherein the aqueous phase is at a concentration by weight of from 80% to 90%, based upon weight of the composition;
(b) dimethicone is present at a concentration by weight of from about 4% to about 20%, based upon weight of the composition; and
(c) the emulsifying crosslinked siloxane elastomer is present at a concentration by weight of from 1% to 5%, based upon weight of the composition.

9. The composition of claim any one of claims 1 to 8, wherein the emulsifying crosslinked siloxane elastomer is selected from the group consisting of dimethicone/copolyol crosspolymer, dimethicone and dimethicone/PEG-10/15
crosspolymers, dimethicone/polyglyceryl crosspolymer, dimethicone and dimethicone/polyglycerin-3 crosspolymer.

10. A process for reducing signs of aging on a keratinous substrate comprising the steps of:
applying the composition of claim 1 onto the keratinous substrate; and
applying shear onto the composition, thereby transforming the composition into a plurality of droplets containing at least one water-soluble ingredient that is forced into the keratinous substrate.

11. The composition of claim 1 for use in the therapy of sunburn or skin cancer development by inhibiting UV rays from contacting a keratinous substrate, the use comprising the steps of:
applying the composition onto a surface of the keratinous substrate; and
applying shear onto the composition, thereby transforming the composition into a plurality of droplets containing the UV sunscreen filter.

## Patentansprüche

1. Zusammensetzung, umfassend:
eine wässrige Phase, beinhaltend mindestens einen wasserlöslichen UV-Sonnenschutzfilter in einer Konzentration nach Gewicht von 2 % bis 25 %, bezogen auf das Gewicht der Zusammensetzung; und
eine Ölphase, bestehend aus:
Dimethicon in einer Konzentration nach Gewicht von 1 % bis 25 %, bezogen auf das Gewicht der Zusammensetzung;
einem emulgierenden vernetzten Siloxanelastomer in einer Konzentration nach Gewicht von 0,1 % bis 20 %, bezogen auf das Gewicht der Zusammensetzung; und
wahlweise einem Co-Emulgator in einer Konzentration nach Gewicht von 0,01 % bis 1 %, bezogen auf das Gewicht der Zusammensetzung;
wobei ein Verhältnis nach Gewicht der wässrigen Phase zu der Ölphase 5,0 bis 9,0 beträgt; und
wobei sich die Zusammensetzung bei Anwendung von Scherung von einer Emulsion in eine Vielzahl von Tröpfchen umwandelt.

2. Zusammensetzung nach Anspruch 1, wobei der UV-Sonnenschutzfilter aus Terephthalylidendikampfersulfonsäure (Ecamsule), Phenylbenzimidazolsulfonsäure (Ensulizole), Benzophenon-4, Aminobenzoesäure (PABA), Kampferbenzalkoniummethosulfat, Methylen-bis-benzotriazolyltetramethylbutylphenol (Bisoctrizole), Dinatriumphenyldibenzimidazoltetrasulfonat (Bisdisulizole-Dinatrium), Trisbiphenyltriazin; ihren Derivaten und entsprechenden Salzen; Naphthalinbisimid-Derivaten und kationischen quartären Cinnamidoamin-Salzen und -Derivaten sowie Mischungen davon ausgewählt ist.

3. Zusammensetzung nach Anspruch 1, wobei die wässrige Phase weiter einen anderen wasserlöslichen aktiven Bestandteil beinhaltet, ausgewählt aus Flavonen, Stilbenoiden, Tanninen, Phenolsäuren, Polyphenolen, Vitaminen, Xanthinen, Ceramiden, Cholesterinen, Sphingosinen, C-Glycosiden, zwitterionischen N-substituierten Aminosulfonsäure-Puffern, Zuckern, Nukleinsäuren, α- und β-Hydroxysäuren, Aminopropyltriethoxysilan, Dihydroxyaceton, botanischen Extrakten, Aminosäuren und Peptiden, ihren Derivaten und Kombinationen davon.

4. Zusammensetzung nach Anspruch 1, wobei die Zusammensetzung weiter mindestens eine hydrotrope Substanz beinhaltet, die aus Nicotinamid, Koffein, Natrium-PCA, Natriumsalicylat, Harnstoff oder Hydroxyethylharnstoff ausgewählt ist.

5. Zusammensetzung nach Anspruch 1, wobei der Co-Emulgator ein Polyoxyalkylen-Copolymer ist.

6. Zusammensetzung nach Anspruch 5, wobei der Co-Emulgator ausgewählt ist aus PEG-10-Dimethicon, PEG-9-Polydimethylsiloxyethyldimethicon und PEG-9, Lauryl-PEG-9-Polydimethylsiloxyethyldimethicon, Dimethicon und PEG/PPG-18/18-Dimethicon und Kombinationen davon.

7. Zusammensetzung nach Anspruch 1, weiter beinhaltend ein Pulver in einer Konzentration nach Gewicht von 0,1 % bis 5 %, bezogen auf das Gewicht der Zusammensetzung.

8. Zusammensetzung nach Anspruch 1, wobei:
a) die wässrige Phase mindestens einen wasserlöslichen UV-Sonnenschutzfilter beinhaltet, ausgewählt aus Terephthalylidendikampfersulfonsäure (Ecamsule), Phenylbenzimidazolsulfonsäure (Ensulizole) und Benzophenon-4 in einer Konzentration nach Gewicht von 4 % bis 10 %, bezogen auf das Gewicht der Zusammensetzung, wobei die wässrige Phase in einer Konzentration nach Gewicht von 80 % bis 90 %, bezogen auf das Gewicht der Zusammensetzung, vorliegt;
b) Dimethicon in einer Konzentration nach Gewicht von etwa 4 % bis etwa 20 %, bezogen auf das Gewicht der Zusammensetzung, vorhanden ist; und
c) das emulgierende vernetzte Siloxanelastomer in einer Konzentration nach Gewicht von 1 % bis 5 %, bezogen auf das Gewicht der Zusammensetzung, vorhanden ist.

9. Zusammensetzung nach einem der Ansprüche 1 bis 8, wobei das emulgierende vernetzte Siloxanelastomer aus der Gruppe ausgewählt ist, die aus Dimethicon/Copolyol-Kreuzpolymer, Dimethicon und Dimethicon/PEG-10/15-Kreuzpolymeren, Dimethicon/Polyglyceryl-Kreuzpolymer, Dimethicon und Dimethicon/Polyglycerin-3-Kreuzpolymer besteht.

10. Verfahren zum Reduzieren von Anzeichen des Alterns auf einem keratinösen Substrat, das die folgenden Schritte umfasst:
Aufbringen der Zusammensetzung nach Anspruch 1 auf das keratinöse Substrat; und
Anwenden von Scherung auf die Zusammensetzung, wodurch die Zusammensetzung in eine Vielzahl von Tröpfchen umgeformt wird, die mindestens einen wasserlöslichen Bestandteil enthalten, der in das keratinöse Substrat gedrückt wird.

11. Zusammensetzung nach Anspruch 1 zur Verwendung bei der Therapie von Sonnenbrand- oder Hautkrebsentwicklung, indem das In-Kontakt-Treten von UV-Strahlen mit einem keratinösen Substrat inhibiert wird, wobei die Verwendung die folgenden Schritte umfasst:
Aufbringen der Zusammensetzung auf eine Oberfläche des keratinösen Substrats; und
Anwenden von Scherung auf die Zusammensetzung, wodurch die Zusammensetzung in eine Vielzahl von Tröpfchen umgeformt wird, die den UV-Sonnenschutzfilter enthalten.

## Revendications

1. Composition, comprenant :
une phase aqueuse comportant au moins un filtre de protection solaire UV soluble dans l'eau à une concentration en poids de 2% à 25% par rapport au poids de la composition ; et
une phase huileuse composée de :
diméthicone à une concentration en poids de 1% à 25% par rapport au poids de la composition ;
un élastomère de siloxane réticulé émulsifiant à une concentration en poids de 0,1% à 20% par rapport au poids de la composition ; et
facultativement un co-émulsifiant à une concentration en poids de 0,01% à 1% par rapport au poids de la composition ;
un rapport en poids de la phase aqueuse à la phase huileuse étant de 5,0 à 9,0 ; et
la composition changeant d'une émulsion à une pluralité de gouttelettes lors de l'application d'un cisaillement.

2. Composition selon la revendication 1, le filtre de protection solaire UV étant choisi parmi l'acide téréphtalylidène dicamphre sulfonique (Ecamsule), l'acide phénylbenzimidazole sulfonique (Ensulizole), le benzophénone-4, l'acide aminobenzoïque (PABA), le méthosulfate camphre de benzalkonium, le méthylène bis-benzotriazolyl tétraméthylbutylphénol (Bisoctrizole), le tétrasulfonate de phényldibenzimidazole disodique (Bisdisulizole disodique), la tris-biphényle triazine ; leurs dérivés et leurs sels correspondants ; les dérivés de bisimide de naphtaline, et les sels quaternaires cationiques de cinnamidoamine et leurs dérivés, et leurs mélanges.

3. Composition selon la revendication 1, la phase aqueuse comprenant en outre un autre ingrédient actif soluble dans l'eau, choisi parmi les flavones, les stilbénoïdes, les tanins, les acides phénoliques, les polyphénols, les vitamines, les xanthines, les céramides, les cholestérols, les sphingosines, les C-glycosides, les tampons d'acide aminosulfonique zwitterionique N-substitué, les sucres, les acides nucléiques, les acides α-hydroxylés et β-hydroxylés, l'aminopropyl triéthoxysilane, le dihydroxyacétone, les extraits botaniques, les acides aminés et les peptides, leurs dérivés et leurs combinaisons.

4. Composition selon la revendication 1, la composition comprenant en outre au moins un hydrotrope choisi parmi le nicotinamide, la caféine, l'acide pyroglutamique de sodium, le salicylate de sodium, l'urée ou l'hydroxyéthylurée.

5. Composition selon la revendication 1, le co-émulsifiant étant un copolymère de polyoxyalkylène.

6. Composition selon la revendication 5, le co-émulsifiant étant choisi parmi la PEG-10 diméthicone, la PEG-9 polydiméthylsiloxyéthyl diméthicone et la PEG-9, la lauryl PEG-9 polydiméthylsiloxyéthyl diméthicone, la diméthicone et la PEG/PPG-18/18 diméthicone et leurs combinaisons.

7. Composition selon la revendication 1, comprenant en outre une poudre à une concentration en poids de 0,1% à 5% par rapport au poids de la composition.

8. Composition selon la revendication 1, dans laquelle :
(a) la phase aqueuse comprend au moins un filtre de protection solaire UV soluble dans l'eau, choisi parmi l'acide téréphtalylidène dicamphre sulfonique (Ecamsule), l'acide phénylbenzimidazole sulfonique (Ensulizole), et le benzophénone-4, à une concentration en poids de 4% à 10% par rapport au poids de la composition, la phase aqueuse étant à une concentration en poids de 80% à 90% par rapport au poids de la composition ;
(b) la diméthicone est présente à une concentration en poids d'environ 4% à environ 20% par rapport au poids de la composition ; et
(c) l'élastomère de siloxane réticulé émulsifiant est présent à une concentration en poids de 1% à 5% par rapport au poids de la composition.

9. Composition selon l'une quelconque des revendications 1 à 8, l'élastomère de siloxane réticulé émulsifiant étant choisi dans le groupe constitué d'un polymère réticulé de diméthicone/copolyol, de polymères réticulés de diméthicone et de diméthicone/PEG-10/15, d'un polymère réticulé de diméthicone/polyglycéryle, d'un polymère réticulé de diméthicone et de diméthicone/polyglycérine-3.

10. Procédé pour la réduction de signes de vieillissement sur un substrat kératinique comprenant les étapes suivantes :
l'application de la composition selon la revendication 1 sur le substrat kératinique ; et
l'application d'un cisaillement sur la composition, transformant ainsi la composition en une pluralité de gouttelettes contenant au moins un ingrédient soluble dans l'eau qui est forcé dans le substrat kératinique.

11. Composition selon la revendication 1 pour l'utilisation dans la thérapie lors du développement d'un coup de soleil ou d'un cancer de la peau en empêchant les rayons UV d'entrer en contact avec un substrat kératinique, l'utilisation comprenant les étapes suivantes :
l'application de la composition sur une surface du substrat kératinique ; et
l'application d'un cisaillement sur la composition, transformant ainsi la composition en une pluralité de gouttelettes contenant le filtre de protection solaire UV.
